## Europäisches Patentamt

(19) **European Patent Office**

**Office européen des brevets**

(11) Publication number: **0 108 387**

**B1**

(12) **EUROPEAN PATENT SPECIFICATION**

(45) Date of publication of patent specification: **16.05.90**

(21) Application number: **83110923.6**

(22) Date of filing: **02.11.83**

(51) Int. Cl.⁵: **C 12 N 15/00,** C 12 P 19/34, C 07 C 233/00, C 12 P 21/00 // C12R1/19

(54) Preparation of recombinant growth releasing factors.

(30) Priority: **04.11.82 US 439168**
**10.01.83 US 456660**

(43) Date of publication of application:
**16.05.84 Bulletin 84/20**

(45) Publication of the grant of the patent:
**16.05.90 Bulletin 90/20**

(84) Designated Contracting States:
**AT BE CH DE FR GB IT LI NL**

(56) References cited:
GB-A-2 007 675

Science, vol.218, no. 4572, 05.11.1982,
Washington D.C. USA R.Guillemin et al:
"Growth Hormone-Releasing Factor from a
Human Pancreatic Tumor that Caused
Acromegaly", pp. 585-587

1985 ATCC Catalogue, pp.72,73

Gene, 2, 1977, pp.95-113

Gene, 5, 1979, pp. 59-76

(73) Proprietor: **F. HOFFMANN-LA ROCHE AG**
**Postfach 3255**
**CH-4002 Basel (CH)**

(72) Inventor: **Bhatt, Ram S.**
**11 River Road**
**Nutley, N.J. (US)**
Inventor: **Collier, Kenneth J.**
**12 Wenonah Avenue**
**Rockaway, N.J. (US)**
Inventor: **Crowl, Robert M.**
**246 Paterson Avenue**
**Little Falls, N.J. (US)**
Inventor: **Poonian, Mohindar S.**
**16 Knoll Place**
**West Caldwell, N.J. (US)**

(74) Representative: **Lederer, Franz, Dr. et al**
**Van der Werth, Lederer & Riederer**
**Patentanwälte Lucile-Grahn-Strasse 22**
**D-8000 München 80 (DE)**

(56) References cited:
**Methods in Enzymology, 68, 1979, pp.482-492**
**Journal of Bacteriology, 141, Jan. 1980, pp. 213-222**

Courier Press, Leamington Spa, England.

**Description**

Recently Guillemin and coworkers at the Salk Institute have reported the isolation, synthesis, and biological activity of a group of related substances they have called growth hormone releasing factor (GRF) [Science, 218, 585-587 (November 5, 1982), see also New York Times, October 29, 1982, at page 1, column 2]. This factor has been sought after for decades by scientists but such search has been, until now, unrewarding due to the minute quantities in which such substance occurs naturally.

The successful isolation of GRF has been due in part to the discovery of the ectopic production of GRF in large amounts by pancreatic tumors, associated with acromegaly. Three forms of GRF derived from the pancreatic tumor have been observed: GRF-44, GRF-40 and GRF-37. GRF-44 comprises the complete amino acid sequence of GRF-40 which is extended at the carboxyl terminus by 4 amino acids. GRF-40 again comprises the complete amino acid sequence of GRF-37 which is extended at the carboxyl terminus by 3 amino acids. Additionally GRF-44 has an amidated carboxy terminus whereas GRF-40 and GRF-37 have a free carboxy group at that terminus.

The amidated form of GRF-44 is apparently the parent molecule and has been indicated to possess the highest biological activity in vitro. However, all three peptides have been found to be virtually equally potent in vivo. It has further been shown that the removal of the amino terminal tyrosine from GRF results in complete loss of bioactivity indicating that the active core of the molecule starts with the amino terminal amino acid.

Growth in animals is believed to be regulated by a cascade of bio-regulatory molecules. Thus, the hypothalamus produces GRF which in turn acts upon the pituitary to cause release of growth hormone. The pituitary is maintained under negative feedback control by somatostatin and insulin growth factor (IGF). GRF has been found to be enormously active, exhibiting an $Ed_{50}$ of approximately 50 fmole/ml or 75 pg/ml and has been found to release micrograms/ml levels of growth hormone in the blood. Thus, GRF can be utilized therapeutically in most of the areas now considered candidates for treatment by growth hormone. Examples of such therapeutic uses include the treatment of pituitary dwarfism, diabetes resulting from abnormalities in growth hormone production, enhancement of wound healing, treatment of burns and retardation on the aging process. Due to its favorable potency compared to growth hormone itself, GRF will have major advantages in the agricultural field as well. Agricultural uses would include, for example, stimulating development of fowl or animals raised for meat so as to allow either marketing at an earlier time or else allow the farmer to produce a larger animal per equal time on feed to present methodology. In addition, GRF would be useful in stimulation of milk production in dairy cows and increasing egg production in chickens.

While GRF in its various forms is of a molecular size which would allow for synthesis by either conventional solid phase or solution phase peptide synthetic methods, it is believed that for economic, large scale production of these therapeutically valuable substances the use of recombinant DNA technology is preferred.

There are examples already known in the art for producing recombinant mammalian peptides utilizing genes synthesized by chemical methods. Thus, for example the production of recombinant human somatostatin in E. coli utilizing a chemically synthesized gene is reported in Science 198, 1956 (December 9, 1977). This gene was fused to the E. coli beta-galactosidase gene on the plasmid pBr 322. Transformation of E. coli with the chimeric plasmid DNA lead to the synthesis of a polypeptide including the sequence of amino acids corresponding to somatostatin. Biologically active somatostatin was specifically cleaved from the chimeric protein by treatment with cyanogen bromide. This procedure is described in greater detail in U.K. Patent Application 2,007,675A. More recently substantially larger synthetic genes have been synthesized and cloned such as, for example, the gene for human leukocyte interferon (Nature 292, 756-761 [1981]).

The present invention relates to a method for the production of recombinant GRF and to the means useful in this method, i.e. to the corresponding bacterial transformants, the expression vehicles and DNA sequences. More precisely the present invention relates to double stranded polydeoxyribonucleotides (DNA sequences) consisting of a structural gene coding for human GRF-OH(44) with the amino acid sequence

```
Tyr-Ala-Asp-Ala-Ile-Phe-Thr-Asn-Ser-Tyr-Arg-Lys-Val-Leu-Gly-Gln-
Leu-Ser-Ala-Arg-Lys-Leu-Leu-Gln-Asp-Ile-Met-Ser-Arg-Gln-Gln-Gly-
Glu-Ser-Asn-Gln-Glu-Arg-Gly-Ala-Arg-Ala-Arg-Leu
```

or for a biologically active fragment thereof with at least the first 37 amino acids and of an EcoR I recognition site at the end coding for the amino terminus of said GRF and a Sal I recognition site at the end coding for the carboxy terminus of said GRF, with structural genes coding for GRF-OH(44), GRF-OH(40) and GRF-OH(37) being preferred. The invention also relates to these DNA sequences operably linked with a DNA sequence (promoter and operator) capable of effecting bacterial expression of said GRF-OH, to

EP 0 108 387 B1

replicable bacterial expression vehicles containing such DNA sequences and to bacteria transformed with such expression vehicles.

The method for the production of a recombinant GRF in accordance with the present invention comprises growing a bacterium transformed with a replicable bacterial expression vehicle containing a DNA sequence consisting of a structural gene coding for human GRF-OH(44) with the amino acid sequence

```
Tyr-Ala-Asp-Ala-Ile-Phe-Thr-Asn-Ser-Tyr-Arg-Lys-Val-Leu-Gly-Gln-
Leu-Ser-Ala-Arg-Lys-Leu-Leu-Gln-Asp-Ile-Met-Ser-Arg-Gln-Gln-Gly-
Glu-Ser-Asn-Gln-Glu-Arg-Gly-Ala-Arg-Ala-Arg-Leu
```

or for a biologically active fragment thereof with at least the first 37 amino acids and of an EcoR I recognition site at the end coding for the amino terminus of said GRF and a Sal I recognition site at the end coding for the carboxy terminus of said GRF which is operably linked with a DNA sequence (promoter and operator) capable of effecting bacterial expression of said GRF-OH, inducing expression of the recombinant gene, lysing the bacterium upon completion of the induction period and separating said GRF from the cellular constituents of said bacterium. Such methods involves

(a) preparing a first series of oligodeoxyribonucleotide fragments which, when joined in the proper sequence, yield a DNA strand coding for the amino acid sequence of GRF;

(b) preparing a second series of oligodeoxyribonucleotide fragments which, when joined in proper sequence, yield a DNA strand complementary to the coding strand;

(c) effecting hydrogen bonding between mutually complementary portions of fragments of the first and second series to form double stranded DNA structures; and

(d) ligate the double-stranded DNA structures to yield the desired GRF structural gene.

The resulting gene codes for one of the above GRF forms.

From the fermentation broth the GRF can be isolated and purified so as to provide the GRF essentially free of bacterial protein thus suitable for use as a therapeutic agent. All process steps can be effected in accordance with methods known per se.

The accompanying drawings illustrate aspects of the preferred embodiments of the invention.

Figure 1. Schematic outline of the process by which a preferred cloning and expression vector (pRC 23) containing the bacteriophage lambda $P_L$ promoter is obtained. This vector is constructed by ligating synthetic oligonucleotides containing a "consensus" ribosome binding site (Scherer et al., Nucleic Acids Research 8, 3895 [1980]) to a 250 bp Bgl II-Hae III fragment containing the $P_L$ promoter and inserting the ligation product into the plasmid pRC 2. Further details concerning the construction of pRC 23 see e.g. European patent application publication No. 89676.

Figure 2. Schematic structure of the nucleotide sequence coding for the amino acid sequence of GRF-OH(44). Also shown is the double-stranded gene with restriction endonuclease recognition sites at either end for insertion into the expression vector.

Figure 3. The coding strand of a synthetic gene coding for the amino acid sequence of GRF-44 is depicted. Additionally, restriction endonuclease recognition sites are shown. The GRF structural gene shown is flanked at both ends by appropriate $P_L$ linker sequence to allow ready insertion into a cloning and expression vector. Note that while the peptide sequence of natural GRF(44) terminates in an amide group, the corresponding recombinant GRF terminates in a free carboxy group and thus recombinant GRF(44) is hereafter designated GRF-OH(44).

Figure 4. A series of 18 oligodeoxyribonucleotide fragments which are employed in the construction of the synthetic structural gene for GRF-OH(44) with $P_L$ linker termini is shown. The fragments which are associated with the prime number represent the complementary gene strand.

Figure 5. Flow chart for the construction of the amino terminal section of the synthetic structural gene for GRF-OH(44). The fragment numbers from Figure 4 are utilized.

Figure 6. Flow chart for the construction of the carboxy terminal section of the synthetic structural gene GRF-OH(44). The fragment numbers from Figure 4 are utilized.

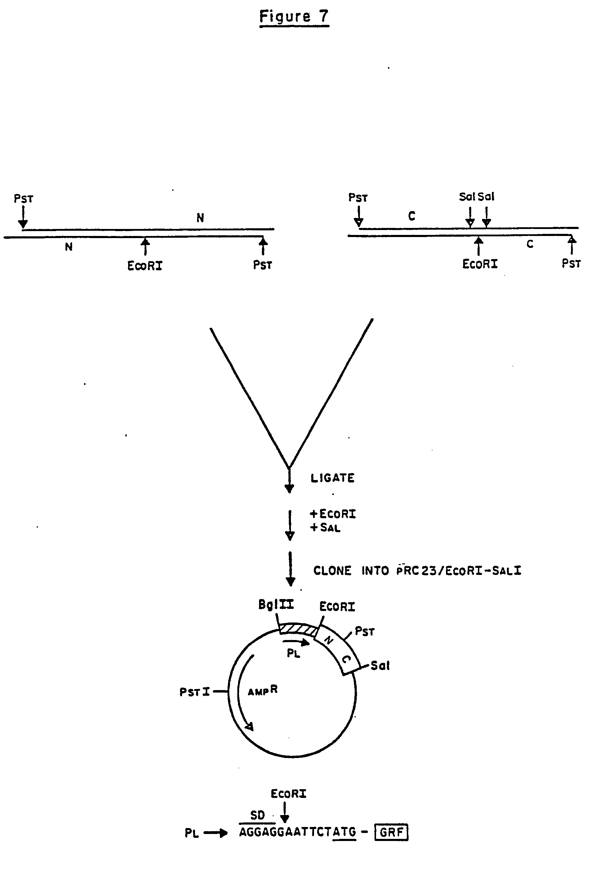

Figure 7. Flow chart for the construction of a recombinant plasmid capable of expressing GRF-OH(44) from parenteral plasmid pRC 23. As seen in this Figure the N (amino terminal) and C (carboxy terminal) fragments of the GRF synthetic gene are first ligated and then inserted into the plasmid. $Amp^R$ denotes the gene for ampicillin resistance. The relative positions of various restriction endonuclease specific cleavage sites on the plasmid are depicted (e.g. Pst I, Bgl II, Eco RI, Sal). Note that the gene for tetracycline resistance ($Tet^R$) normally found on pRC 23 is eliminated by the restriction cut at the Sal site. Thus the recombinant plasmid exhibits only ampicillin resistance for screening.

Figure 8. Schematic outline of the loading of the glass support procedure utilized in the solid support phosphite methodology employed in the synthesis of oligonucleotides 1, 4, 5, 9, 11, 15 and 20. The procedure is adapted from Matteucci and Caruthers, J. Am. Chem. Soc. 103, 3185 (1981).

Figure 9. Schematic outline of the oligonucleotide synthesis scheme using the solid support phosphite method.

3

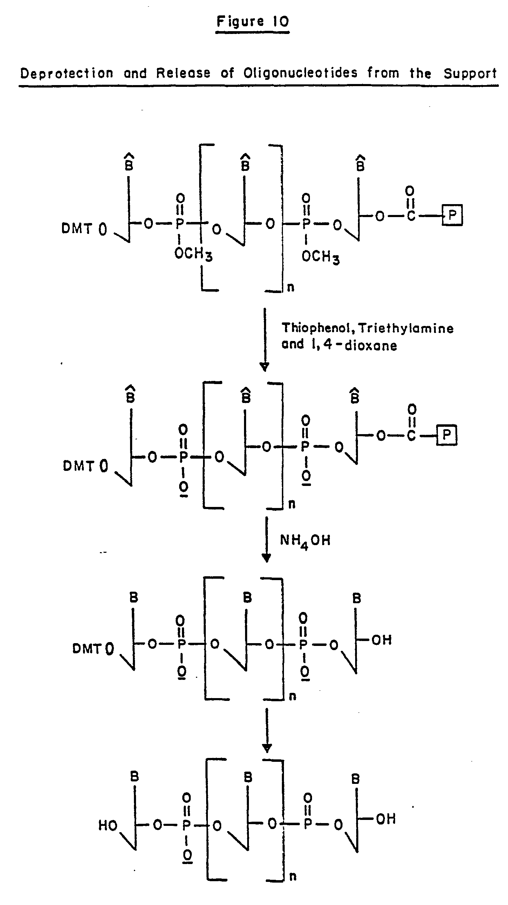

Figure 10. Schematic outline of the deprotection and release of oligonucleotides from the solid support in the solid support phosphite method.

Figure 11. Generic representation of the functional intermediates used in the solid support triester methodology employed for the preparation of fragments 2, 3, 6, 7, 8, 10, 12, 13, 14, 16 and 17. The procedure is adapted from Dembek et al., J. Am. Chem. Soc. 103, 706 (1981).

Figure 12. Schematic outline of the reaction scheme employed in the construction of oligonucleotide fragments by the solid support triester method.

Figure 13. Sequences 14', 15' and 16' for substitution of oligonucleotide fragments 14-18 (Fig. 6) and partial construction of a gene segment (ligation point indicated by arrow) containing these fragments, which unit can be ligated to gene segment 1-13 to form a structural gene for production of GRF-OH(37).

Figure 14. Sequences 14'', 15'' and 16'' for substitution of oligonucleotide fragments 14-18 (Fig. 6) and partial construction of a gene segment (ligation point indicated by arrow) containing these fragments, which unit can be ligated to gene segment 1-13 to form a structural gene for production of GRF-OH(40).

Figure 15. Flow chart for the construction of a recombinant plasmid capable of expressing GRF-OH(44) using monomeric N and C fragments.

Detailed Description of the Invention

Preparation of Genes coding for GRF

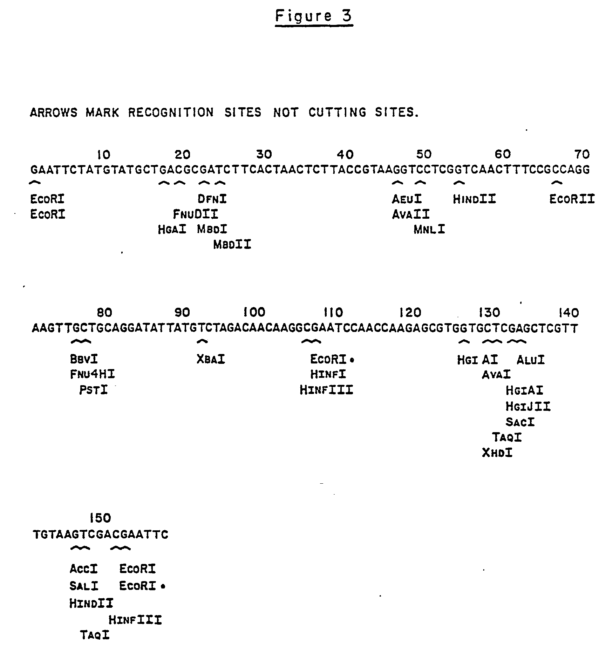

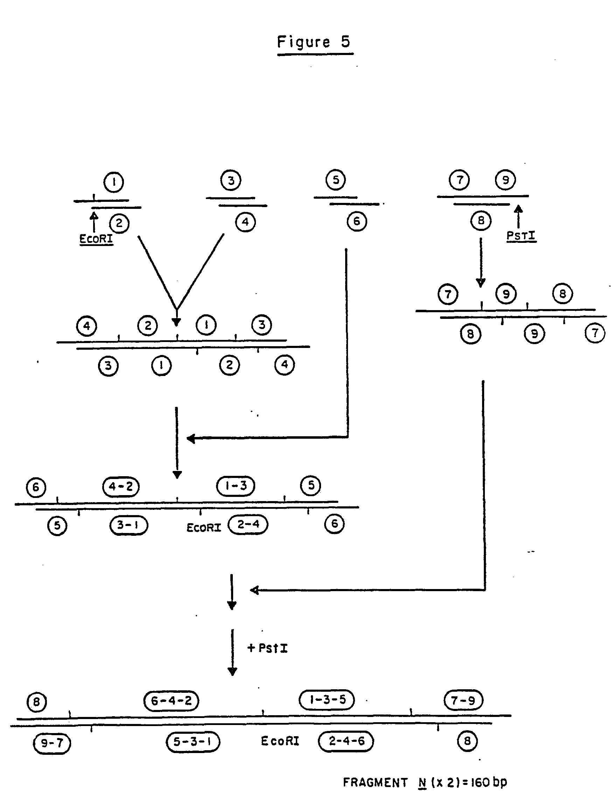

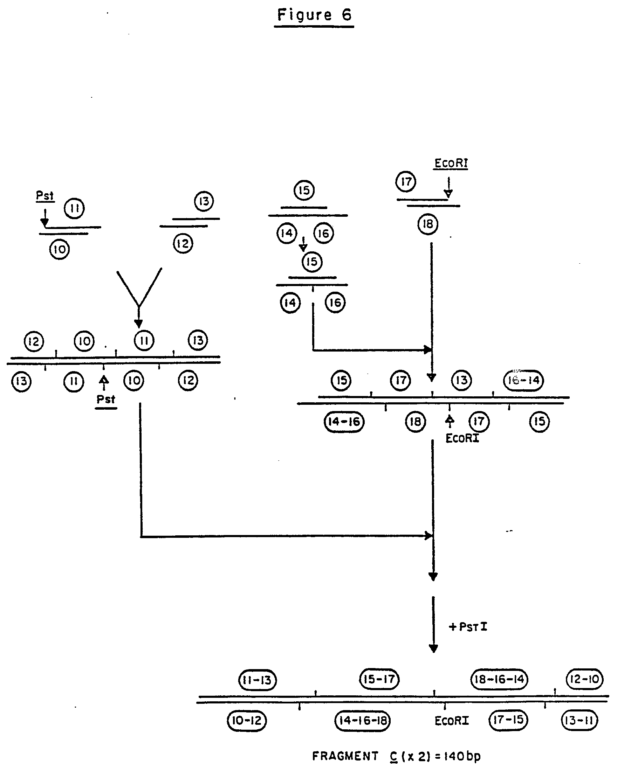

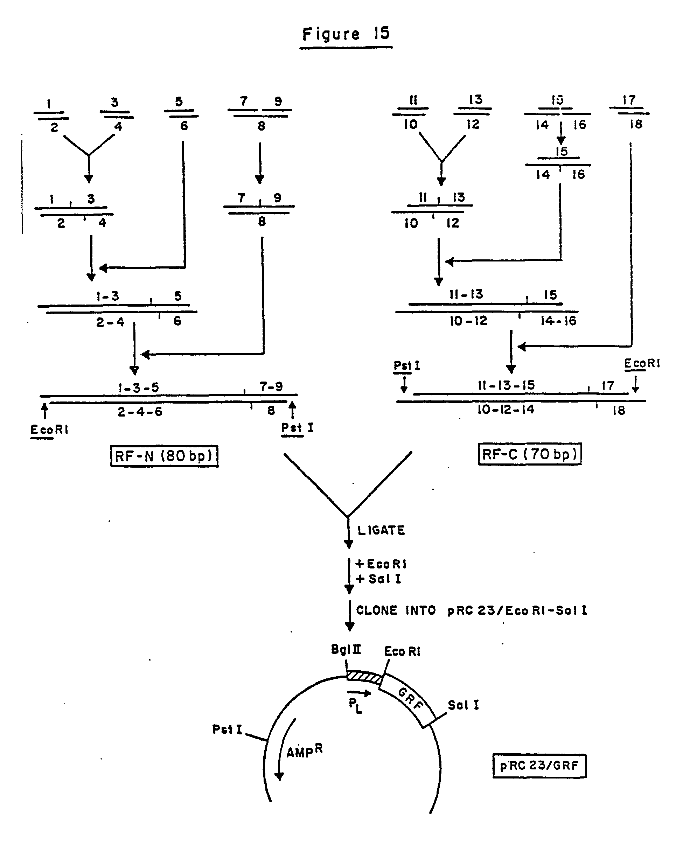

DNA coding for any desired GRF, i.e. GRF-OH(44), GRF-OH(40) and GRF-OH(37), may be prepared by chosing codons according to the genetic code. For ease in preparation and purification, oligodeoxyribonucleotide fragments of, for example, from about 14 to about 20 nucleotides are prepared separately, and assembled to provide the desired sequences. Thus, one prepares a first and second series of oligodeoxyribonucleotide fragments of convenient size. The first series, when joined in proper sequence, yields a DNA strand coding for GRF (see Figure 3, fragments 1, 3, 5, 7, 9, 11, 13, 15 and 17). The second series when likewise joined in proper sequence, yields a strand complementary to the coding strand (see Figure 3, fragments 2, 4, 6, 8, 10, 12, 14, 16 and 18). The fragments of the coding and the complementary strand preferably overlap such that their self assembly promotes through hydrogen bonding of the cohesive termini of fragment blocks. Following assembly, preferably in the two-step approach illustrated in Figures 5 and 6, for dimer N and C fragments or, more preferably, for monomer N and C fragments as illustrated in Figure 15, the structural gene is completed by ligation in the conventional manner.

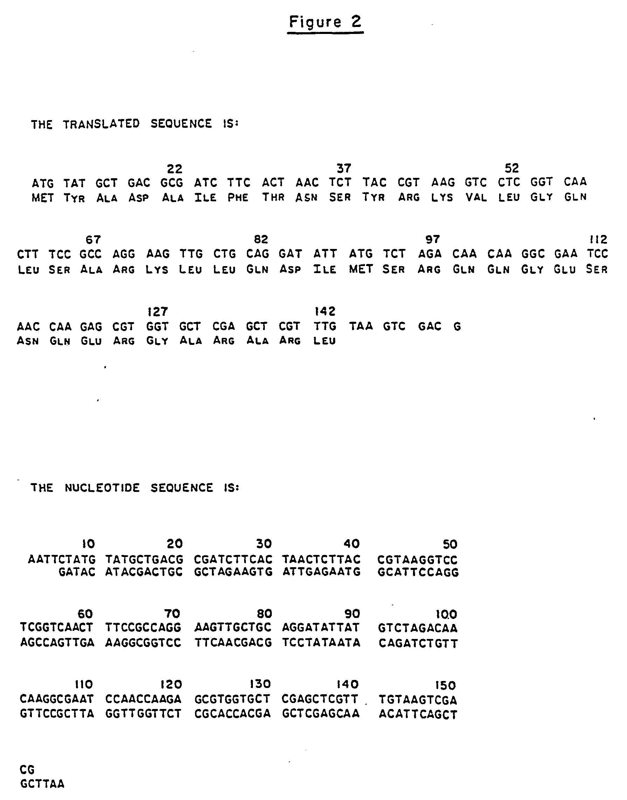

The degeneracy of the genetic code permits substantial freedom in the choice of codons for any given amino acid sequence. See for example, Figure 2. For present purposes however, codons preferred in the expression of microbial genomes have been used advantageously.

Thus the E. coli condon preferences were judiciously incorporated with codons for yeast expression so that the genes will be suitable for bacterial and yeast expression vectors. The gene sequence selected also provides convenient restriction sites which facilitate assembly of the gene and its subsequent analysis. The selected sequence for GRF was computer-scanned to ensure the absence of self-complementary and repeated sequences which might interfere with proper ligation of the oligonucleotides during assembly.

For expression in E. coli, the coding sequence for GRF is preceded by an ATG initiation codon and followed by a single TAA translational stop codon. EcoRI termini were placed at the beginning and at the end of the gene for convenient insertion into the expression vector(s). A Sal I site was also introduced following the coding sequence as an alternative site for insertion into the expression vector and to facilitate rapid Maxam-Gilbert sequencing of the gene once it is cloned.

Preparation of the Expression Vector

Preferred expression vectors used in the practice of the present invention comprise hybrid ribosome binding sites. Ribosome binding sites (RBS) from which the hybrid RBS are derived, comprise RNA sequences encoded by DNA. RBS's are necessary for the initiation of translation in a host cell. RBS's consist essentially of (1) the ATG codon initiating translation (all known E. coli gene products begin with the amino acid methionine which may be may not be subsequently cleaved off); (2) A sequence of 3 to 9 bases which are complementary to bases at the 3' end of 16S ribosomal RNA known as Shine-Dalgarno (SD) sequence (Shine, J. and Dalgarno, L., Nature 254, 34 [1975]); and (3) a sequence of bases between these two sequences known as linker region.

The expression vectors forming the preferred embodiment of this invention are derivatives of plasmid pBR 322 containing the $P_L$ promoter isolated from bacteriophage lambda DNA and inserted between the $tet^R$ and the $amp^R$ genes. $P_L$ is a promoter of choice since it is a very strong promoter that can be efficiently and conveniently controlled by the lambda cl repressor. The gene encoding the repressor carries a mutation, clts2 or clts857, which renders the repressor temperature-sensitive. At 30°C the repressor functions normally, and from about 37-42°C it is inactivated. Thus, the $P_L$ promoter is repressed (turned-off) at 30°C and derepressed (turned-on) at 42°C. The ability to control the $p_L$ promoter allows for the growth of the culture at about 30-36°C without expression of the gene product and to shift to the production of the desired GRF product by raising the temperature to about 37-42°C.

The preferred vector used in the present invention also contains an EcoRI restriction site distal (downstream in the 3' direction) of the SD sequence, providing a means of constructing different hybrid RBS's. Once the hybrid RBS is constructed using the EcoRI site to join the SD sequence to the ATG start coding of the GRF structural gene, it can be further modified by restriction with EcoRI, filling-in the termini with Klenow Polymerase I and joining the two resulting blunt-ends by ligation with $T_4$ DNA ligase.

It is also within the skill of the art to utilize other control elements to produce alternative expression vectors in conjunction with the novel structural genes for GRF provided by the present invention. Obviously some modification may be required in the termini provided to allow insertion of the gene into the requisite plasmids. Suitable systems for use in bacteria according to the broadest aspects of this invention include the lac promoter-operator system, the arabinose operon (phi 80 dara) or the colicine EI, galactose, alkaline phosphatase or tryptophan operons. Similarly the ADH system can be employed to provide expression in yeast.

The Microorganism

A large number of unicellular microorganisms are known in the art as being suitable for transformation. Particular microorganisms include bacteria, fungi, and algae. Preferred organisms for transformation include bacteria such as strains of E. coli; Bacillaceae, such as Bacillus subtilis and the like. Yeasts form a further preferred group of microorganisms for transformation.

In a preferred embodiment of this invention, the microorganism employed as the recipient in the transformation procedures is E. coli K-12 strain 294 as described in British Patent Publication No. 2055382A. Strains of this microorganism have been deposited with the American Type Culture Collection, ATCC Accession Nos. 31446 and 31448 deposited October 28, 1978 and are publicly available. Other suitable E. coli strains may also be employed, such as E. coli MA 210 or RBI.

The present invention is further illustrated by the following Examples.

## Example 1

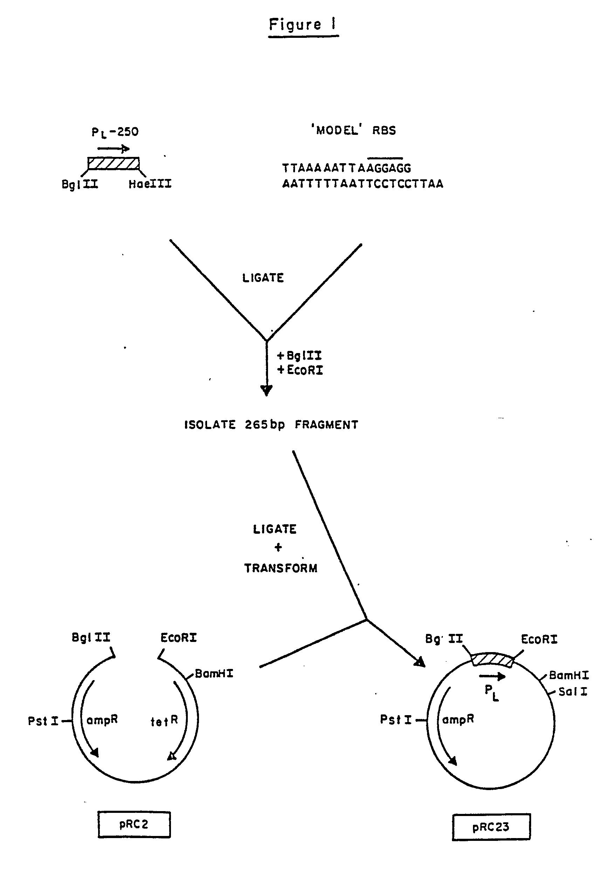

pRC23 was constructed by ligating synthetic oligonucleotides comprising a "consensus" RBS [Scherrer, et al. Nucleic Acids Research, 8, 3895 (1980)] to a 250 bp Bgl II — Hae III fragment containing the lambda $P_L$ promoter, and inserting the ligation product into pRC2 as shown in Figure 1. The details of this construction are described below.

In order to isolate the 250 base pair (bp) DNA fragment containing the lambda $P_L$ promoter, 1 µg of a 450 bp Bgl II — Hpa I DNA fragment (from bp 35260 to 35710 of the November 1981 version of the lambda DNA sequence) was digested with Hae III and the products were isolated by preparative gel electrophoresis in 5% polyacrylamide. About 200 µg of the 250 bp Bgl II — Hae III fragment was ligated to 60 pmoles each of the synthetic oligonucleotides shown in Figure 1 which comprise most of the "consensus" ribosome-binding site sequences generated by computer analysis as described by Scherer et al. in Nucleic Acids Research 8, 3895 (1980). The ligated molecules were digested with Bgl II and EcoRI (to eliminate oligomers) and purified by gel electrophoresis. The ligated products were then ligated into pRC2 which also had been digested with Bgl II and EcoRI. pRC2 is a derivative of pBR322 with a Bgl II site located adjacent to the EcoRI site (see Figure 1). Transformation of E. coli RRI (pRK248clts) was performed using standard methods and transformants were selected on media containing ampicillin (50 µg/ml) at 30°C. 50 transformants were obtained, DNA was isolated from 8 of those and analyzed by digesting with Hinc II. 6 of the 8 showed the expected restriction pattern and Maxam-Gilbert nucleotide sequence analysis of one of these confirmed the expected construction (designated pRC23).

## Example 2
### Standard Method for Preparation of 3'-O-Succinylnucleosides

The 5'-O-dimethoxytrityl and N-protected deoxynucleoside (2.5 mmole) is dissolved in dry pyridine (5 ml) and N,N-dimethylaminopyridine (0.3 g). Succinic anhydride (2.0 mmole, 0.2 g) is added and the solution stirred at room temperature for 24 hrs. After the reaction is completed, solvent is evaporated under vacuum and the dry gum is coevaporated with toluene twice (10 ml each time). The residue is dissolved in methylene chloride (30 ml) and the solution extracted with citric acid (ice cold). The organic phase is washed twice with water (15 ml each) and then dried over anhydrous sodium sulfate. In order to avoid any detritylation of the product, about 0.3 ml of pyridine is added to the methylene chloride solution. The methylene chloride solution is concentrated to about 10 ml and the succinylated nucleoside is isolated by precipitation into hexane:ether (1:1, v/v; 250 ml). The precipitate is collected by filtration and dried in vacuo.

## Example 3
### Standard Method for Preparation of p-Nitrophenyl Esters of Succinylated Nucleosides

Succinylated nucleoside (1 mmole) is dissolved in dry dioxane (3 ml) containing pyridine (0.3 ml). to the above solution is added dicyclohexylcarbodiimide (DCC) (5 mmole, 900 mg) and p-nitrophenol (0.22 g, 1 mmole). The solution is stirred for 2 hrs. The precipitate of dicyclohexylurea is removed by centrifugation. The supernatant is directly used for coupling to the resin as described in the next Example.

## Example 4
### Condensation of Succinylated Nucleosides to the Resin for Solid Support Phosphite Methodology

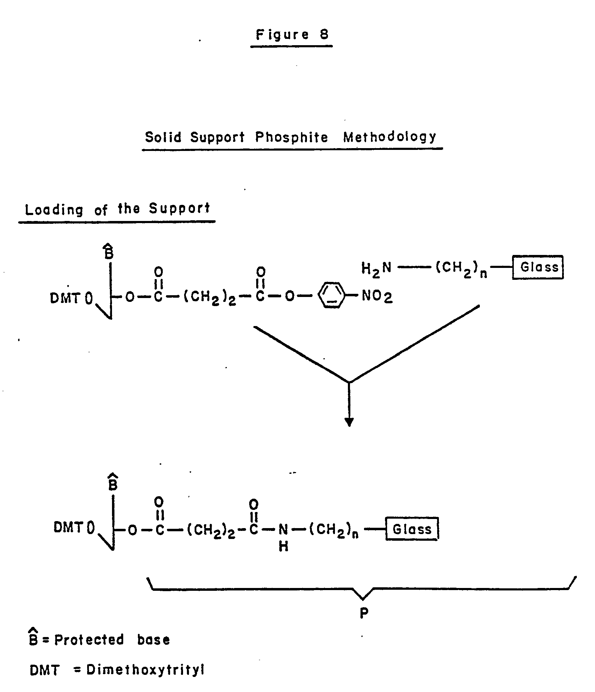

A 25 ml (~10 g) sample of Pierce Long Chain Alkylamine/CPG (controlled pore glass) support (pore

diameter 500 Å, particle size 125—177 μ) is suspended in 25 ml dry dimethylformamide (DMF). The solution containing the p-nitrophenyl ester of the succinylated nucleoside is added to the above suspension (Fig. 8). The contents are shaken for 24 hrs. The derivatized resin is isolated, washed with dry DMF (3 times, 30 ml each), dioxane (5 times, 30 ml each), methanol (5 times, 30 ml each) and finally with anhydrous ether (3 times, 30 ml each). Any unreacted amino functions are blocked by reacting the support with a solution of dry pyridine (25 ml), N,N-dimethylamino pyridine (250 mg) and acetic anhydride (5 ml) for 2 hrs. at room temperature. The solid support is filtered and washed successively with methanol (5 times, 30 ml) and anhydrous ether (2 times, 30 ml).

Approximately 1 mg quantity of the nucleoside-loaded resin is treated with 0.1 M toluenesulfonic acid in acetonitrile (1 ml). The trityl carbonium ion released from the resin as a red-orange color is collected and diluted appropriately with acetonitrile to measure optical density at 498 nm. An average of 4 such measurements provided the extent of loading on the support. The range of loading obtained from several different resin loading experiments was 22-25 μmole of nucleoside/g of the solid support.

Example 5

Standard Preparation of 5'-O-Dimethoxytrityl-3'-O-phosphoroamidite Derivatives of Nucleosides

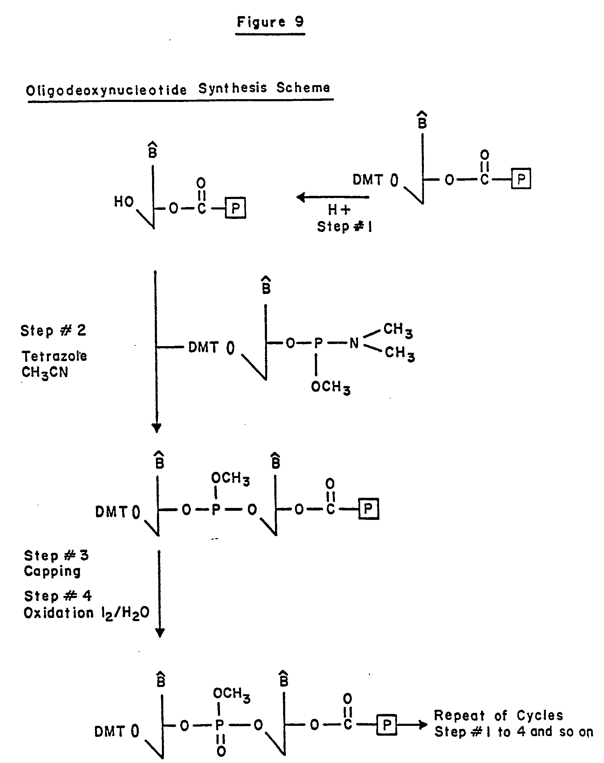

As shown in Figure 9, following the procedure of Beaucage and Caruthers, Tetrahedron Letters *22*, 1859 (1981), a sample of 5'-O-dimethoxytrityl nucleoside (5 mmole) is dissolved in dry and acid-free chloroform. To the solution is added diisopropylethyl-amine (2.74 ml, 15.74 mmole) followed by a gradual addition of dimethylamino-methyl phosphonomonochloridite ($CH_3OP(Cl)N(CH_3)_2$) over 1-2 minutes. The reaction mixture is transferred to a separatory funnel with 175 ml of ethyl acetate. The organic mixture is extracted with saturated brine (4 times, 75 ml). The ethyl acetate phase is dried over anhydrous sodium sulfate. The solution is concentrated to an oil and then redissolved in dry ethyl acetate to obtain 50 ml of the solution. This solution is added dropwise to precooled (ice-acetone temperature) n-hexane (250 ml) for precipitation of the product which is then filtered and dried under high vacuum for 17 hrs. at room temperature. For all four nucleoside derivatives yields of ~80% have been achieved. The samples of the dimethylamino phosphoroamidites were stored under argon at −14°C.

Example 6

Solid Support Synthesis of Fragment No. 1 5'-AATTCTATGTATGCTGA-3' by Phosphite Methodology

Step 1: A sample of 5'-O-dimethoxytrityl-deoxyadenosine-loaded glass support (435 mg, 10 μmole of dimethoxytrityl-deoxyadenosine/g of support) was detritylated with 0.2 M dichloroacetic acid in dichloromethane (2 ml) by shaking the suspension for 1.5 min. The support was washed with dichloromethane (2 times, 5 ml). A repeat of dichloroacetic acid treatment for a few seconds was given to insure completion of detritylation.

The support was washed successively:
a) with 1% triethylamine in dichloromethane (2 times, 5 ml)
b) with anhydrous acetonitrile (9 times, 3 ml).

Step 2: A solution of dimethylaminophosphoroamidite derivative of dimethoxytrityl-deoxyguanosine (100 μmole in 2 ml anhydrous acetonitrile) was added to the support followed by the addition of tetrazole solution (250 μmole in 2 ml of acetonitrile). The suspension was shaken for 5 minutes, vacuum filtered and washed with anhydrous acetonitrile (4 × 5 ml).

Step 3: A 2 ml solution of acetic anhydride (0.4 ml) in pyridine containing about 10 mg of N,N-dimethylaminopyridine was shaken with the support for 2 minutes to block any unreacted hydroxyl functions and then the blocked support was washed with acetonitrile (4 times, 5 ml).

Step 4: A solution of iodine (0.2 M) in tetrahydrofuran/water/2,6-lutidine (2:1:1 v/v) was shaken with the blocked support for 0.5 min., washed with dry acetonitrile (6 times, 5 ml) and washed with dichloromethane (2 times, 5 ml).

The Step 1 is repeated with the product of Step 4; Step 2 is repeated with the phosphoroamidite derivative of 5'-O-dimethoxytrityl thymidine and finally Steps 3 and 4 are repeated.

The cycle (Steps 1 through 4) is then repeated over and over with the addition of each successive nucleoside derivative, thus extending the chain in the 5'-direction by adding corresponding phosphoramide derivatives of deoxycytidine (dC), deoxyguanosine (dG), thymidine (T), deoxyadenosine (dA), T, dG, T, dA, T, dC, T, T, dA and dA respectively to yield the desired fragment.

Example 7

Deprotection and Release of the Oligonucleotides from the Support

A sample of the oligonucleotide-bound support obtained in Example 6 (150 mg) was treated with a mixture of 1,4-dioxane (0.25 ml), 0.25 ml anhydrous triethylamine and 0.125 ml thiophenol for 30 minutes at room temperature. The resin was filtered, washed with methanol (4 × 2 ml) and hydrolyzed with concentrated ammonium hydroxide for 17 hours at 50°C. The resin was pelleted and the supernatant concentrated under vacuum and redissolved in water (1 ml). The solution of the oligonucleotides was passed through a 0.45 micron filter to remove very fine suspension and then fractionated on a high performance liquid chromatographic (HPLC) system using a C 18 reverse phase Microbondapak (Waters Assoc.) column under a gradient of 10-30% acetonitrile in 0.05 M triethylammonium acetate, pH 7.0, at 50°C. The well separated dimethoxytrityl containing product was collected, concentrated and treated with 80% aqueous acetic acid for 0.5 hr at room temperature. After evaporation of acetic acid, the sample was dissolved in water and extracted with ether to remove trityl alcohol. The aqueous layer containing the desired oligonucleotide was separated and examined for its purity on HPLC. In several cases a second purification at this stage was found necessary to obtain homogeneous product. Final criterium of purity was analysis of the 5'-$^{32}$P-labeled oligonucleotide on acrylamide gel electrophoresis under denaturing conditions. The nucleotide sequence of the fragments was ascertained by the Maxam-Gilbert method, Proc. Nat. Acad. Sciences (USA) 74, 560 (1977).

Following the same approach as described above, synthesis of fragments 4, 5, 9, 11, 15 and 18 is accomplished.

Example 8

Standard Method For Phosphotriester Solid-Phase Synthesis of Heptadecanucleotide d(CGCGATCTTCACTAACT), Fragment 3 of GRF Gene

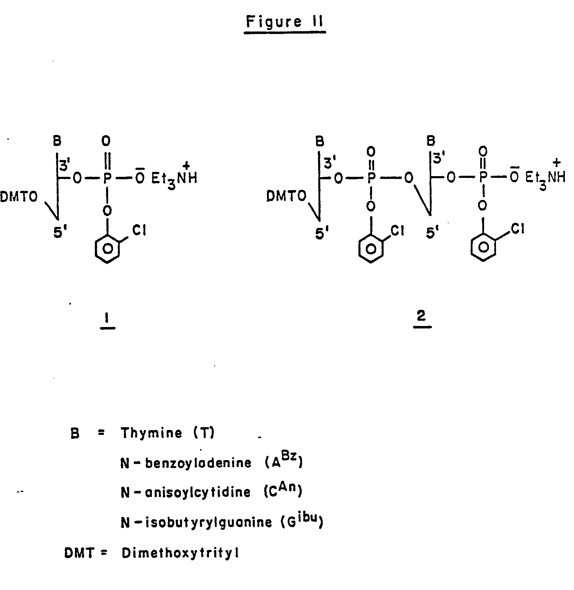

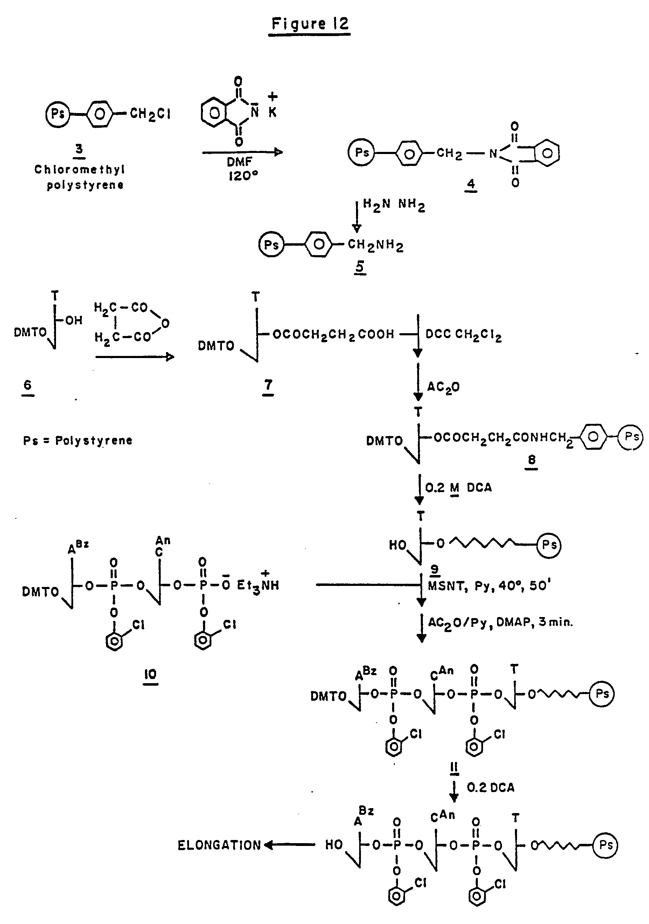

The preparation of fragment 3 of the GRF gene by the phosphotriester solid-phase method was achieved according to the Scheme outlined. The numbers of compounds refer to Figures 11 and 12. The reaction steps are:

(a) Synthesis of suitably blocked mono- and dinucleotides of the general structure 1 and 2 respectively.

(b) Preparation of aminomethyl polystyrene resin 5.

(c) Loading of succinate 7 on to the aminomethyl polystyrene resin 5 and masking of any unreacted amino group with acetic anhydride-pyridine.

(d) Removal of 5'-dimethoxytrityl group of polymer supported nucleoside 8 with 0.2 M dichloroacetic acid (DCA) in CH$_2$Cl$_2$ to afford a 5'-hydroxy function (resin 9) for the coupling reaction.

(e) Condensation of the triethylammonium salt of the dimer 10 with resin 9 to provide polymer supported trinucleotide 11. Any unreacted 5'-OH group from 9 is masked with 10% acetic anhydride in pyridine.

(f) Detritylation of 11 with 0.2 M DCA to generate a new 5'-OH group for the next coupling.

The aforesaid suitably protected mono-dinucleotides were synthesized according to procedures known in the art with slight modification. 3'-succinate 7 of 5'-dimethoxytrityl-thymidine 6 was prepared according to the method published by Miyoshi et al., Nucleic acids Research 8, 5491 (1980).

Aminomethyl Polystyrene Resin 5:

A slurry of commercially available chloromethyl polystyrene (30 g, 9 mmole, 1% cross linked, 0.32 mmole/g Cl$^-$) and potassium phthalimide (2.77 g, 15 mmole) in 250 ml DMF was heated at 120°C for 20 hours. The resin was then filtered while hot and washed consecutively with hot DMF (200 ml), water (4 × 50 ml), dioxane (3 × 80 ml), ethanol (3 × 80 ml) and ether (3 × 50 ml) to give the phthalimide derivative 4 which was suspended in ethanol (350 ml) and refluxed with hydrazine (5 ml) for 5 hrs. The reaction mixture was filtered and washed with hot ethanol (4 × 70 ml), hot DMF (2 × 70 ml), water (3 × 70 ml), ethanol (3 × 70 ml) and ether (3 × 80 ml). The resin was then dried (28 g) to give aminomethyl polystyrene 5. Picric acid titration showed amino group concentration of 0.21 mmole/g.

Loading of 5'-Dimethoxy-3'-O-succinyl-thymidine 7 on to Aminomethyl Resin 5:

To a slurry of aminomethyl resin 5 (5 g) in dry CH$_2$Cl$_2$ (30 ml) was added monosuccinate 7 (0.97 g, 1.5 mmole), DCC (0.927 g) and dimethylaminopyridine (DMAP) (50 mg) and the reaction mixture was allowed to stir at room temperature for 4 hrs. After filtering, resin 8 was washed with CH$_2$Cl$_2$ (4 × 15 ml), MeOH (4 × 15 ml), CH$_2$Cl$_2$ (4 × 15 ml),) and ether (3 × 10 ml). After drying resin 8 under vacuum (0.1 mm Hg), the concentration of the loaded nucleoside was determined by measuring the absorption spectrum of the liberated tritanol from the support in a 1% BSA solution of CHCl$_3$ (λ max 498, ε 92100] and was found to be 0.185 mmole/g.

Masking of the unreacted amino group from resin 5, along with resin 8, was achieved by reacting the entire mixture of the resins with 10% acetic anhydride-pyridine mixture (30 ml) containing dimethylaminopyridine (DMAP) (30 mg) for 30 minutes at room temperature. The resin was then washed with CH$_2$CL$_2$, MeOH, CH$_2$Cl$_2$ and ether. The concentration of the loaded nucleoside was again determined as described above and was found to be 0.178 mmole/g.

Detritylation of Resin 8 with 0.2 M Dichloroacetic acid in CH$_2$Cl$_2$:

Table I below shows the conditions used for detritylation of each of the dimethoxytrityl groups of each of the four nucleosides. A typical experiment is as follows: the resin was first swollen in dry CH$_2$Cl$_2$ (2 ml) for about 30 seconds in a small column (1.3 × 10 cm) and then shaken with 10 ml of 0.2 M DCA for 30—40 seconds followed by a very quick filtration and very quick washed with CH$_2$Cl$_2$ (3-4 ml). This process was repeated (see Table I) until the washings did not show any color due to tritanol. The washings were collected and the absorption spectrum measured to determine the concentration of the tritanol evolved which, in turn, reflects the concentration of the loaded mono- or dinucleotide.

After complete detritylation had been achieved, resin 9 was washed with 0.5 M Et$_3$N solution in CH$_2$Cl$_2$ and then with CH$_2$Cl$_2$ (3 × 10 ml) and pyridine (3 × 5 ml).

## Table I

| N of (DMT)N-P | Number of Operation with 0.2 M DCA | Time for each operation | Washings with $CH_2Cl_2$ |
|---|---|---|---|
| T | 5 x 10 ml | 40 sec. | 1 x 3 ml |
| C | 5 x 10 ml | 30 sec. | 1 x 3 ml |
| G | 3 x 10 ml | 30 sec. | 1 x 3 ml |
| A | 4 x 10 ml | 20 sec. | 1 x 3 ml |

Coupling of AC dimer 10 with 5'-hydroxy-polystyrene support 9 to give the polymer-supported ACT trimer 11:

The 5'-hydroxy resin 9 (57 mg, 0.01 mmole) which had been washed with pyridine (see above) was dried under high vacuum (0.05 mm Hg) and by blowing hot air on the column from a heat gun for 2 minutes. The vacuum was carefully released by letting in dry air or argon. To the resin was then added a pyridine (0.7 ml) solution of the triethylammonium salt 10 (0.06 mmole) of the AC dimer followed by the addition of mesitylenesulfonyl-3-nitro-triazole (MSNT) (0.18 mmole). The column was properly stoppered and heated at 40°C for 50 min. The reaction mixture was filtered and washed thrice with pyridine (3 ml each time). Unreacted 5'-OH groups were masked by reacting the resin with 10% Ac$_2$O/Py containing DMAP (10 mg) for 3 minutes at room temperature. The resin was filtered and successively washed with pyridine (2 × 2 ml),CH$_2$Cl$_2$ (2 × 5 ml), MeOH (2 × 5 ml) and again with CH$_2$Cl$_2$ (2 × 5 ml) to afford the ACT trimer 11 supported by polystyrene polymer.

The 5'-dimethoxytrityl group of resin 11 was removed with 0.2 M DCA as described above (Table I) and 5'-hydroxy resin 12 resulting therefrom was extended to the desired heptadecanucleotide by repeating each cycle with a dimer or a monomer of choice. The average coupling yield estimated by the absorption spectrum of the tritanol liberated from the support was 94%.

Deblocking and Purification:

After the last coupling reaction, the resin (50 mg) was treated with a 0.5 molar solution of $N^1,N^1,N^3,N^3$-tetramethylguanidinium pyridine-2-carboxyaldoximate (1 ml) in 80% (v/v) dioxane-H$_2$O for 15 hours at room temperature according to the procedure of Reese et al., Tet. Letters, *19*, 2727 (1978). The solvent was then evaporated off, the residue left was treated with concentrated ammonia (28%, 4 ml) at 60°C for 8 hrs. After centrifugation, the supernatant was concentrated to a volume of ca. 0.5 ml which was dissolved in 0.05 M TEAB (1.5 ml) and passed through a Sephadex G-50 column using 0.05 M TEAB as a mobile phase. The eluted fractions (6 ml each) were collected and the absorption was measured on a Beckman UV spectrophotometer Model 34. Fractions 30-33 containing the product were collected to give 100.8 ODs (OD = optical density). 50 ODs out of this product were purified by HPLC on a Microbondapak C$_{18}$ column using a linear gradient of 7.5 to 37.5% acetonitrile (pH 7.8) over a period of 12 minutes. Peak 2 eluting with 31% CH$_3$CN, contained the dimethoxytrityl product and was collected to give 30.2 ODs. The solvent was evaporated off, the residue left was detritylated with 80% acetic acid (20 minutes at room temperature). Acetic acid was evaporated off and the residue left was coevaporated with toluene (3 × 1 ml) to remove residual acetic acid. The resulting residue was dissolved in water (2 ml), and extracted with ether (3 × 3 ml) to remove any organic impurities. The aqueous solution was carefully concentrated to a volume of 400 µl, filtered and purified on a Microbondapak C$_{18}$ HPLC column using a linear gradient of 7.5-37.5% acetonitrile. The peak eluting with 20.6% acetonitrile was collected and further purified by electrophoresis on an acrylamide gel in the presence of 7 M urea. The slowest moving band was isolated by buffer elution and after labelling the 5'-hydroxy group with γ[$^{32}$P]-ATP, the sequence of the heptadecanucleotide fragment 3, was confirmed by Maxam-Gilbert sequence analysis.

Following the same approach as described above, synthesis of fragments 2, 6, 7, 8, 10, 12, 13, 14, 16 and 17 is accomplished.

## Example 9

### Dimer Construction

As outlined in Figures 5 and 6, the oligonucleotides are assembled in two stages. Oligonucleotides Nos. 1-9 are ligated following the scheme of Figure 5 to generate fragment N, the amino-terminal half of the GRF gene. Likewise, oligonucleotides Nos. 10-18 are ligated to generate fragment C, the carboxy-terminal half of the gene as seen in Figure 6. Dimers of N and C are cloned individually into the Pst I site of PBr 322 (see Bolivar et al., Gene 2, 95 [1977]), to take advantage of insectional inactivation of the beta-lactamase gene as an initial screen of the transformants. Plasmid DNA from transformants showing a tet$^R$ amp$^S$ phenotyop are further analyzed to confirm that the correct fragments are cloned. After amplification of the plasmid DNA carrying N and C, the fragments are excised by digesting with Pst I and purified by preparative gel electrophoresis. The dimers of N and C are then ligated together and digested with EcoRI and Sal I to generate the monomeric form of the completely assembled GRF gene (see Figure 7).

Specific procedures for the aforesaid ligations are set forth below:

300 pmoles of each of the oligonucleotides (Nos. 1-18) are phosphorylated with T$_4$ polynucleotide kinase in 20 µl reaction mixtures containing 50 mM Tris-HCl (pH 7.4), 10 mM MgCl$_2$, 0.1 mM Na$_3$EDTA, 5 mM DTT, 0.1 mM spermidine-HCl, and a 5-fold molar excess of ATP (γ-$^{32}$P-ATP) at 37°C for 30 minutes.

Equimolar amounts of each oligonucleotide are ligated under standard conditions (50 mM Tris, pH 7.4, 10 mM MgCl$_2$, 10 mM DTT, 0.3 mM ATP) with 4 units of T$_4$ DNA ligase at 15°C for 3-12 hours. Ligation reactions are monitored by gel electrophoresis in 20% polyacrylamide, and where necessary, the ligation products are purified by preparative gel electrophoresis.

### Monomer Construction

As outlined in Figure 15, the oligonucleotides were assembled in two stages. Oligonucleotides Nos. 1-9 were ligated following the scheme in Figure 15 to generate fragment RF-N, the amino-terminal half of the GRF gene. Likewise, oligonucleotides Nos. 10-18 were ligated to generate fragment RF-C, the carboxy-terminal half of the gene as seen in Figure 15.

Specific procedures for the aforesaid ligations are set forth below:

20 pmoles each of the synthetic fragments Nos. 1-18 were phosphorylated with 5 units of polynucleotide kinase in 10 µl reaction mixture containing 50 mM Tris-HCl (pH 7.4), 10 mM MgCl$_2$, 0.1 mM EDTA, 5 mM DTT, 0.1 mM spermidine-HCl 12.5 M ATP, and 37.5 µC [γ-$^{32}$P] ATP (carrier-free) at 37°C for 30 minutes. The reactions were terminated by heating at 65°C for 5 min. followed by the addition of 5 µl of 0.5 M EDTA, 5 µl of 10 mg/ml tRNA, and 190 µl of 5 M ammonium acetate. The nucleic acids were ethanol precipitated, resuspended in 200 µl of 0.3 M sodium acetate, ethanol precipitated again, dried under a vacuum and resuspended in 10 µl of 1 mM Tris (pH 7.4), 0.1 mM EDTA (= 0.1 × TE).

The two halves of the GRF gene (RF-N and RF-C) were assembled separately following the schemes outlined in Figure 15. 8 pmoles each of the phosphorylated oligonucleotides were ligated with 4 units of T4 DNA ligase in reaction mixtures containing 50 mM Tris (pH 7.4), 10 mM MgCl$_2$, 10 mM DTT, and 0.3 mM ATP at 15°C for 30 to 60 minutes. At the end of the sequential ligations, and additional 200 units of ligase was added and incubation was continued at 4°C for 2 days. The ligation reactions were terminated by heating at 65°C for 5 min., then chilled on ice. The ligated molecules were digested with PstI and EcoRI, ethanol precipitated, and separated by electrophoresis on a 8% polyacrylamide gel. The 80 bp product comprising the N-terminal portion of the GRF gene, and the 70 bp product comprising the C-terminal portion were identified following autoradiography, recovered from the gel, ethanol precipitated twice, dried, and resuspended in 10 µl of 0.1 × TE.

## Example 10

For direct expression of the GRF gene product in E. coli, the EcoRI-Sal I fragment containing the GRF gene is inserted into the expression vector pRC23 which has been digested with EcoRI and Sal I. Joining the GRF sequence at this EcoRI site creates a very favorable ribosome-binding site for efficient translation initiation. Transcription initiation from the strong P$_L$ promoter is conveniently and efficiently controlled by the temperature-sensitive cI repressor encoded by the low-copy number plasmid pRK248clts.

Specific procedures for constructing the aforesaid recombinant plasmid using the monomer construction from Example 9 are set forth below:

3 µl of the purified RF-N and RF-C fragments were ligated together in a 10 µl reaction mixture as described in Example 9 with 200 units of T4 DNA ligase at 15°C for 15 hrs. The reaction was terminated by heating at 65°C for 5 min. and the mixture chilled on ice. The ligated molecules were digested with EcoRI and Sal I, and then ligated as before to 200 ng of the vector pRC23 (which has also been digested with EcoRI and Sal I). The ligation mixture was used to transform E. coli strain RRI (pRK248clts) following standard procedures. Transformants were selected on media containing ampicillin (50 µg/ml). Approximately 200 transformants were obtained. Plasmid DNA was isolated from 10 of the transformants and analyzed by digestion with Ava I (which cuts once within the GRF gene and once in pRC23). 7 out of the 10 showed the expected restriction pattern. Maxam-Gilbert nucleotide sequence analysis of one of these confirmed the expected sequence of the entire GRF gene as shown in Figure 2. This transformant was designated RRI(pRK248clts, pRC23/GRF-1).

To test for expression of the cloned synthetic GRF gene, two approaches were taken: 1. Analysis of the cell-free translation products encoded by pRC23/GRF-1. 2. Assay of lysates of induced cells containing pRC23/GRF-1 for GRF bio-activity. The details of these experiments are described below.

1. Purified pRC23/GRF-1 DNA (along with control DNA) was added to a coupled in vitro transcriptiontranslation system [Kung et al., Arch. Biochem. Biophys. 195, 396 (1979)] containing $^{35}$S-methionine and the resulting products were analyzed by SDS-polyacrylamide gel electrophoresis followed by fluorography. The results indicated that pRC23/GRF-1 directs the sythesis of a polypeptide of 5—6 kilodaltons (authentic GRF-44 is about 5.3 Kd) that is not produced by the control pRC23 DNA. Parallel reactions (carried out with unlabeled methionine) were tested for bioactivity as described below.

2. Strain RRI (pRK248clts, pRC23/GRF) was grown in M9-glucose medium at 30°C to about $2 \times 10^8$ cells/ml, then induced at 42°C for 2 hrs. 1 ml samples were taken and the cells were collected by centrifugation. The cells were resuspended in 50 µl of 50 mM Tris (pH 7.4). 10% sucrose and quickly frozen in a dry ice/ethanol bath. The cells were thawed and kept at 0—4°C. 5 µl of lysis mix (5 mM Nacl, 0.5 M EDTA, 1 M spermidine-HCl, and 5 mg/ml lysozyme, 1:1:1:2, v/v) was added, and after 30 min. on ice the mixture was incubated at 37°C for 2 min. Lysis was complete after an additional cycle of freezing and thawing. Cell debris was removed by centrifugation and the lysates were quickly frozen in a dry ice/ethanol bath. The lysates together with the cell-free translation products described above were assayed for GRF biological activity as described by Brazeau et al., Regul. Peptides 1, 255 (1981). The results shown in Table 2 demonstrate that the synthetic gene for GRF cloned in the expression vector pRC23 directs the synthesis of biologically active GRF polypeptide.

## Table 2

| Sample Number | Plasmid | GRF Activity Percent of Control |
|---|---|---|
| 1 | Control (media only) | 100 |
| 2 | Control + 25 fmoles of GRF-NH$_2$ (44) | 320 |
| 3 | pRC23 + 25 fmoles of GRF-NH$_2$ (44) | 109* |
| 4 | pRC23/IFN-γ | 65 |
| 5 | pRC23/GRF-1 | 96 |
| 6 | pRC23/GRF-9 | 97 |
| 7 | pRC23 | 47 |
| 8 | pRC23/IFN-γ | 59 |
| 9 | pRC23/GRF-9 | 104 |

Samples Nos 3-6 were lysates from induced cells containing the indicated plasmids as described above. Samples Nos. 7-9 were reaction mixtures from cell-free transcription/translation of purified plasmids as indicated.

* A comparison of No. 3 indicates that the bacterial lysate inhibits GRF activity. Therefore, although the data for the GRF samples (Nos. 5, 6 and 9) are not above background (No. 1), they are significantly higher than the negative controls (Nos. 4, 7 and 8) and comparable to the sample with purified synthetic GRF-NH$_2$ (44) added to the bacterial lysate.

The designation pRC23/IFN-γ represents an expression vector containing the human immune interferon gene. This construction was included in the present assay as a negative control as was the parent vector pRC23.

Purification of the GRF polypeptide from the lysate can be accomplished using chromatographic procedures well known in the art. Such procedures include affinity chromatography employing supported GRF polyclonal or monoclonal antibody, column chromatography or, preferably one or more reverse phase HPLC procedures and can be used in combination with each other. In this manner recombinant GRF-OH (44) is obtained in homogeneous form suitable for therapeutic use.

## Example 11

GRF-OH(37) Gene:

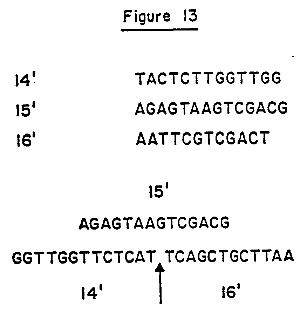

DNA fragments Nos. 1 to 13 used for the construction of GRF-OH(44) gene are also used for the GRF-OH(37) gene. Additionally the fragments 14', 15' and 16' (Fig. 13) are synthesized by the phosphite solid support methodology described hereinbefore and are ligated to the partial structural gene of GRF-OH(44) (built up by fragments Nos. 1-13) to produce the structural gene for GRF-OH(37) as shown in Figure 13.

## Example 12

GRF-OH(40):

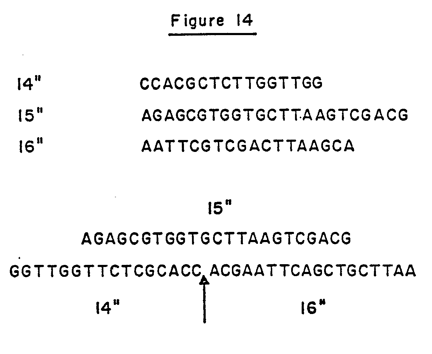

DNA fragments Nos. 1 to 13 used for the construction of GRF-OH(44) gene are also used for the GRF-OH(40) gene. Additionally the fragments Nos. 14'', 15'' and 16'' (Fig. 14) which are synthesized by the triester solid support methodology as hereinbefore described are ligated to the partial structural gene of GRF-OH(44) (built up by fragments Nos. 1-13) to produce the structural gene for GRF-OH(40) as shown in Fig. 14.

Cloning and expression of the GRF-OH(37) and GRF-OH(40) genes may be carried out according to the same procedures described above for the GRF-OH(44) gene.

**Claims for the Contracting States: BE CH DE FR GB IT LI LU NL SE**

1. A double-stranded polydeoxyribonucleotide consisting of a structural gene coding for human GRF-OH(44) with the amino acid sequence

Tyr-Ala-Asp-Ala-Ile-Phe-Thr-Asn-Ser-Tyr-Arg-Lys-Val-Leu-Gly-Gln-

Leu-Ser-Ala-Arg-Lys-Leu-Leu-Gln-Asp-Ile-Met-Ser-Arg-Gln-Gln-Gly-

Glu-Ser-Asn-Gln-Glu-Arg-Gly-Ala-Arg-Ala-Arg-Leu

or for a biologically active fragment thereof with at least the first 37 amino acids and of an EcoR I recognition site at the end coding for the amino terminus of said GRF and a Sal I recognition site at the end coding for the carboxy terminus of said GRF.

2. A polydeoxyribonucleotide according to claim 1 wherein the structural gene codes for human GRF-OH(44).

3. A polydeoxyribonucleotide according to claim 1 wherein the structural gene codes for human GRF-OG(40).

4. A polydeoxyribonucleotide according to claim 1 wherein the structural gene codes for huamn GRF-OH(37).

5. A DNA sequence as claimed in any one of claims 1-4 operably linked with a DNA sequence (promoter and operator) capable of effecting bacterial expression of said GRF-OH.

6. A replicable bacterial expression vehicle containing a DNA sequence as claimed in claim 5.

7. The expression vehicle of claim 6 comprising a promoter and operator derived from bacteriophage lambda and a hybrid ribosome binding site.

8. The expression vehicle of claim 7, wherein the promoter and operator are $P_L$ and $O_L$.

9. The expression vehicle of claim 7 which is plasmid pRC23 into which after cleavage with EcoR I and Sal I has been inserted a DNA sequence according to claim 1.

10. The expression vehicle of claim 9 wherein the structural gene codes for GRF-OH(44).

11. The expression vehicle of claim 9 wherein the structural gene codes for GRF-OH(40).

12. The expression vehicle of claim 9 wherein the structural gene codes for GRF-OH(37).

13. A bacterium transformed with the expression vehicle as claimed in any one of claims 6-12.

14. A bacterium according to claim 13 which is an E. coli strain.

15. An E. coli strain transformed with an expression vehicle as claimed in claim 10.

16. An E. coli strain transformed with an expression vehicle as claimed in claim 11.

17. An E. coli strain transformed with an expression vehicle as claimed in claim 12.

18. A method for the production of a recombinant GRF comprising growing a bacterium transformed with an expression vehicle as claimed in any one of claims 6-12, inducing expression of the recombinant gene, lysing the bacterium upon completion of the induction period and separating said GRF from the cellular constituents of said bacterium.

19. The method of claim 18 wherein said bacterium is grown at a temperature in the range of about 30 to 36°C and gene expression is induced by raising the temperature to about 42°C.

20. The method of claim 19 wherein said recombinant GRF is GRF-OH(44).

21. The method of claim 19 wherein said recombinant GRF is GRF-OH(40).

22. The method of claim 19 wherein said recombinant GRF is GRF-OH(37).

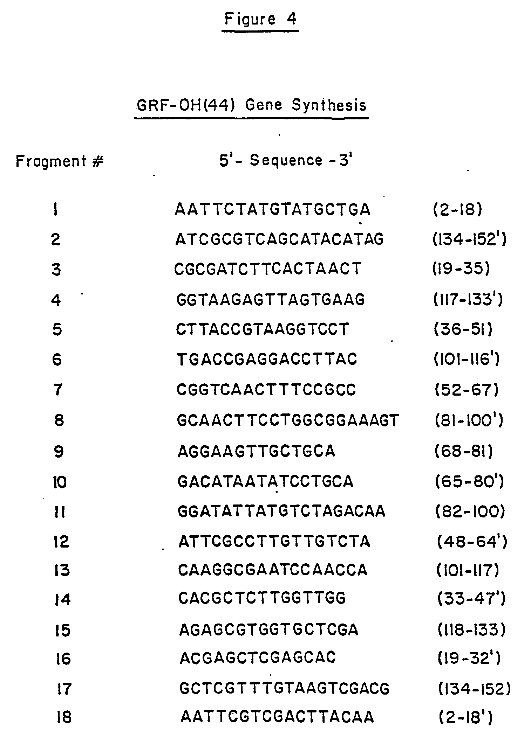

23. A structural gene coding for GRF-OH(44) of the DNA sequence set forth in Figure 2 and obtained by ligating fragments 1-18 of Figure 4 as defined hereinbefore.

24. A structural gene coding for GRF-OH(40) of the sequence obtained by ligating the gene segment set forth in Figure 14 formed from fragments 14'', 15'' and 16'' with the gene segment obtained by ligating fragments 1-13 of Figure 4 as defined hereinbefore.

25. A structural gene coding for GRF-OH(37) of the DNA sequence obtained by ligating the gene

11

segment set forth in Figure 13 formed from fragments 14', 15' and 16' with the gene segment obtained by ligating fragments 1-13 of Figure 4 as defined hereinbefore.

**Claims for the Contracting State: AT**

1. A method for the production of a recombinant GRF comprising growing a bacterium transformed with a replicable bacterial expression vehicle containing a DNA sequence consisting of a structural gene coding for human GRF-OH(44) with the amino acid sequence

Tyr-Ala-Asp-Ala-Ile-Phe-Thr-Asn-Ser-Tyr-Arg-Lys-Val-Leu-Gly-Gln-

Leu-Ser-Ala-Arg-Lys-Leu-Leu-Gln-Asp-Ile-Met-Ser-Arg-Gln-Gln-Gly-

Glu-Ser-Asn-Gln-Glu-Arg-Gly-Ala-Arg-Ala-Arg-Leu

or for a biologically active fragment thereof with at least the first 37 amino acids and of an EcoR I recognition site at the end coding for the amino terminus of said GRF and a Sal I recognition site at the end coding for the carboxy terminus of said GRF which is operably linked with a DNA sequence (promoter and operator) capable of effecting bacterial expression of said GRF-OH, inducing expression of the recombinant gene, lysing the bacterium upon completion of the induction period and separating said GRF from the cellular constituents of said bacterium.

2. The method according to claim 1 wherein the structural gene codes for human GRF-OH(44).

3. The method according to claim 1 wherein the structural gene codes for human GRF-OG(40).

4. The method according to claim 1 wherein the structural gene codes for human GRF-OH(37).

5. The method of any one of claims 1-4 with a promoter and operator derived from bacteriophage lambda and a hybrid ribosome binding site.

6. The method of claim 5 wherein the promoter and operator are $P_L$ and $O_L$.

7. The method of claim 6 wherein the replicable bacterial expression vehicle is plasmid pRC23 into which after cleavage with EcoR I and Sal I has been inserted the structural gene according to claim 1.

8. The method of any one of claims 1-7 wherein the bacterium is an E. coli strain.

9. The method of any one of claims 1-8 wherein said bacterium is grown at a temperature in the range of about 30 to 36°C and gene expression is induced by raising the temperature to about 42°C.

**Patentansprüche für die Vertragsstaaten: BE CH DE FR GB IT LI LU NL SE**

1. Ein doppelsträngiges Polydeoxyribonukleotid, das aus einem Strukturgen, das für Human-GRF-OH(44) mit der Aminosäuresequenz

Tyr-Ala-Asp-Ala-Ile-Phe-Thr-Asn-Ser-Tyr-Arg-Lys-Val-Leu-Gly-Gln-

Leu-Ser-Ala-Arg-Lys-Leu-Leu-Gln-Asp-Ile-Met-Ser-Arg-Gln-Gln-Gly-

Glu-Ser-Asn-Gln-Glu-Arg-Gly-Ala-Arg-Ala-Arg-Leu

oder für ein biologisch aktives Fragment davon mit wenigstens den ersten 37 Aminosäuren kodiert, und aus einer EcoRI-Erkennungsstelle am Ende, die für den Aminoterminus besagten GRFs kodiert, und aus einer SalI-Erkennungsstelle am Ende, die für den Carboxyterminus besagten GRFs kodiert, besteht.

2. Ein Polydeoxyribonukleotid gemäss Anspruch 1, worin das Strukturgen für Human-GRF-OH(44) kodiert.

3. Ein Polydeoxyribonukleotid gemäss Anspruch 1, worin das Strukturgen für Human-GRF-OH(40) kodiert.

4. Ein Polydeoxyribonukleotid gemäss Anspruch 1, worin das Strukturgen für Human-GRF-OH(37) kodiert.

5. Eine DNA-Sequenz, wie in einem jeden der Ansprüche 1-4 beansprucht, die in einer zur Durchführung geeigneten Weise mit einer DNA-Sequenz (Promotor und Operator), die fähig ist, die bakterielle Expression besagten GRF-OHs zu bewirken, verbunden ist.

6. Ein replizierbars baktierielles Expressionsvehikel das eine wie in Anspruch 5 beanspruchte DNA-Sequenz enthält.

7. Das Expressionsvehikel von Anspruch 6 das einen vom Bakteriophagen Lambda abgeleiteten Promotor und einen Operator und eine hybride Ribosomenbindungsstelle enthält.

8. Das Expressionsvehikel von Anspruch 7, worin der Promoter $P_L$ und der Operator $O_L$ ist.

9. Das Expressionsvehikel von Anspruch 7, welches das Plasmid pRC23 ist, in das nach Spaltung mit EcoRI und SalI eine DNA-Sequenz gemäss Anspruch 1 eingefügt worden ist.

10. Das Expressionsvehikel von Anspruch 9, worin das Strukturgen für GRF-OH(44) kodiert.

11. Das Expressionsvehikel von Anspruch 9, worin das Strukturgen für GRF-OH(40) kodiert.

12. Das Expressionsvehikel von Anspruch 9, worin das Strukturgen für GRF-OH(37) kodiert.

13. Ein Bakterium das mit einem wie in einem jeden der Ansprüche 6-12 beanspruchten Expressionsvehikel transformiert worden ist.

14. Ein Bakterium gemäss Anspruch 13 das ein Stamm von E. coli ist.

15. Ein mit einem wie in Anspruch 10 beanspruchten Expressionsvehikel transformierter E. coli Stamm.

16. Ein mit einem wie in Anspruch 11 beanspruchten Expressionsvehikel transformierter E. coli Stamm.

17. Ein mit einem wie in Anspruch 12 beanspruchten Expressionsvehikel transformierter E. coli Stamm.

18. Ein Verfahren zur Herstellung von rekombinantem GRF das daraus besteht, dass man ein mit einem wie in einem jeden der Ansprüche 6—12 beanspruchten Expressionsvehikel transformiertes Bakterium wachsen lässt, die Expression des rekombinanten Gens induziert, die Bakterien nach vollständiger Induktionszeit lysiert und besagtes GRF von den zellulären Bestandteilen besagten Bakteriums abtrennt.

19. Das Verfahren von Anspruch 18, worin man besagtes Bakterium bei einer Temperatur in dem Bereich von etwa 30 bis 36°C wachsen lässt und die Genexpression durch Anheben der Temperatur auf etwa 42°C induziert.

20. Das Verfahren von Anspruch 19, worin besagtes rekombinantes GRF GRF-OH(44) ist.

21. Das Verfahren von Anspruch 19, worin besagtes rekombinantes GRF GRF-OH(40) ist.

22. Das Verfahren von Anspruch 19, worin besagtes rekombinantes GRF GRF-OH(37) ist.

23. Ein Strukturgen das für GRF-OH(44) der in Abbildung 2 dargestellten und wie hier vorstehend definiert durch Verbinden der Fragmente 1-18 aus Abbildung 4 erhaltenen DNA-Sequenz kodiert.

24. Ein Strukturgen das für GRF-OH(40) kodiert, dessen Sequenz durch Verbinden des in Abbildung 14 dargestellten und aus den Fragmenten "14", "15" und "16" gebildeten Gensegments mit dem durch wie hier vorstehend definiert durch Verbinden der Fragmente 1-13 aus Abbildung 4 erhaltenen Gensegment erhalten wurde.

25. Ein Strukturgen das für GRF-OH(37) kodiert, dessen DNA-Sequenz durch Verbinden des in Abbildung 13 dargestellten und aus den Fragmenten "14", "15" und "16" gebildeten Gensegments mit dem durch wie hier vorstehend definiert durch Verbinden der Fragmente 1-13 aus Abbildung 4 erhaltenen Gensegment erhalten wurde.

**Patentansprüche für den Vertragsstaat: AT**

1. Ein Verfahren zur Herstellung eines rekombinanten GRF das daraus besteht, dass man ein Bakterium wachsen lässt, das mit einem replizierbaren bakteriellen Expressionsvehikel transformiert wurde, welches eine DNA-Sequenz enthält, welche aus einem Strukturgen, das für Human GRF-OH(44) mit der Aminosäuresequenz

```
Tyr-Ala-Asp-Ala-Ile-Phe-Thr-Asn-Ser-Tyr-Arg-Lys-Val-Leu-Gly-Gln-

Leu-Ser-Ala-Arg-Lys-Leu-Leu-Gln-Asp-Ile-Met-Ser-Arg-Gln-Gln-Gly-

Glu-Ser-Asn-Gln-Glu-Arg-Gly-Ala-Arg-Ala-Arg-Leu
```

oder ein biologisch aktives Fragment davon mit wenigstens den ersten 37 Aminosäuren kodiert, und aus einer EcoRI-Erkennungsstelle am Ende, die für den Aminoterminus besagten GRFs kodiert, und aus einer SalI-Erkennungsstelle am Ende, die für den Carboxyterminus besagten GRFs kodiert, besteht, welche in einer zur Durchführung geeigneten Weise mit einer DNA-Sequenz (Promotor und Operator), die fähig ist, die bakterielle Expression besagten GRF-OHs zu bewirken, verbunden ist, die Expression des rekombinanten Gens induziert, die Bakterien nach Vervollständigung des Induktionszeitraums lysiert und besagtes GRF von den zellulären Bestandteilen besagten Bakteriums abtrennt.

2. Ein Verfahren gemäss Anspruch 1, worin das Strukturgen für Human-GRF-OH(44) kodiert.

3. Ein Verfahren gemäss Anspruch 1, worin das Strukturgen für Human-GRF-OH(40) kodiert.

4. Ein Verfahren gemäss Anspruch 1, worin das Strukturgen für Human-GRF-OH(37) kodiert.

5. Ein Verfahren gemäss jedem der Ansprüche 1-4 mit einem Promotor und einem Operator die von dem Bakteriophagen Lambda abgeleitet sind und einer hybriden Ribosomenbindungsstelle.

6. Das Verfahren von Anspruch 5, worin der Promotor $P_L$ und der Operator $O_L$ ist.

7. Das Verfahren von Anspruch 6, worin das replizierbare bakterielle Expressionsvehikel das Plasmid pRC23 ist in das nach Spaltung mit EcoRI und SalI das Strukturgen gemäss Anspruch 1 eingefügt worden ist.

8. Das Verfahren eines jeden der Ansprüche 1-7, worin das Bakterium ein E. coli Stamm ist.

9. Das Verfahren eines jeden der Ansprüche 1-8, worin man das besagte Bakterium bei einer Temperatur im Bereich von etwa 30-36°C wachsen lässt und die Genexpression durch Erhöhen der Temperatur auf etwa 42°C induziert wird.

# EP 0 108 387 B1

**Revendications pour les Etats contractants: BE CH DE FR GB IT LI LU NL SE**

1. Polydésoxyribonucléotide double-brin consistant en un gène de structure codant pour GRF-OH(44) humain avec la séquence d'acides aminés

```
Tyr-Ala-Asp-Ala-Ile-Phe-Thr-Asn-Ser-Tyr-Arg-Lys-Val-Leu-Gly-Gln-
Leu-Ser-Ala-Arg-Lys-Leu-Leu-Gln-Asp-Ile-Met-Ser-Arg-Gln-Gln-Gly-
Glu-Ser-Asn-Gln-Glu-Arg-Gly-Ala-Arg-Ala-Arg-Leu
```

ou pour un fragment biologiquement actif de celui-ci avec au moins les 37 premiers acides aminés et en un site de reconnaissance EcorR I à l'extrémité codant pour l'extrémité amino terminale dudit GRF et un site de reconnaissance Sal I à l'extrémité codant pour l'extrémité carboxy terminale dudit GRF.

2. Polydésoxyribonucléotide selon la revendication 1 ou le gène de structure code pour GRF-OH(44) humain.

3. Polydésoxyribonucléotide selon la revendication 1 ou le gène de structure code pour GRF-OH(40) humain.

4. Polydésoxyribonucléotide selon la revendication 1 ou le gène de structure code pour GRF-OH(37) humain.

5. Séquence d'ADN selon l'une quelconque des revendications 1 à 4 liée de façon opérationnelle à une séquence d'ADN (promoteur et opérateur) capable d'effectuer l'expression bactérienne dudit GRF-OH.

6. Vecteur d'expression bactérienne réplicable contenant une séquence d'ADN selon la revendication 5.

7. Le vecteur d'expression de la revendication 6, comprenant un promoteur et un opérateur dérivés du bactériophage lambda et un site de liaison ribosomique hybride.

8. Le vecteur d'expression de la revendication 7 dans lequel le promoteur et l'opérateur sont $P_L$ et $O_L$.

9. Le vecteur d'expression de la revendication 7 qui est le plasmide pRC23 dans lequel, après clivage par EcoR I et Sal I, a été insérée une séquence d'ADN selon la revendication 1.

10. Le vecteur d'expression de la revendication 9 où le gène de structure code pour GRF-OH(44).

11. Le vecteur d'expression de la revendication 9 où le gène de structure code pour GRF-OH(40).

12. Le vecteur d'expression de la revendication 9 où le gène de structure code pour GRF-OH(37).

13. Bactérie transformée par le vecteur d'expression selon l'une quelconque des revendications 6 à 12.

14. Bactérie selon la revendication 13, qui est une souche de E. coli.

15. Souche de E. coli transformée par un vecteur d'expression selon la revendication 10.

16. Souche de E. coli transformée par un vecteur d'expression selon la revendication 11.

17. Souche de E. coli transformée par un vecteur d'expression selon la revendication 12.

18. Méthode pour la production d'un GRF recombinant comprenant la croissance d'une bactérie transformée par un vecteur d'expression selon l'une quelconque des revendications 6 à 12, l'induction de l'expression du gène recombinant, la lyse de la bactérie à la fin de la période d'induction et la séparation dudit GRF à partir des constituants cellulaires de ladite bactérie.

19. La méthode de la revendication 18 dans laquelle ladite bactérie est cultivée à une température comprise entre environ 30°C et 36°C et le gène d'expression est induit en élevant la température à environ 42°C.

20. La méthode de la revendication 19 dans laquelle ledit GRF recombinant est GRF-OH(44).

21. La méthode de la revendication 19 dans laquelle ledit GRF recombinant est GRF-OH(40).

22. La méthode de la revendication 19 dans laquelle le GRF recombinant est GRF-OH(37).

23. Gène de structure codant pour GRF-OH(44) de la séquence d'ADN représentée à la figure 2 et obtenue par ligature des fragments 1-18 de la figure 4 comme défini précédemment.

24. Gène de structure codant pour GRF-OH(40) de la séquence obtenue par ligature du segment de gène représenté à la figure 14 formé à partir des fragments 14'', 15'' et 16'' avec le segment de gène obtenu par ligature des fragments 1-13 de la figure 4 comme défini précédemment.

25. Gène de structure codant pour GRF-OH(37) de la séquence d'ADN obtenue par ligature du segment de gène représenté à la figure 13 formé à partir des fragments 14', 15' et 16' avec le segment de gène obtenu par ligature des fragments 1-13 de la figure 4 comme défini précédemment.

**Revendications pour l'Etat contractant: AT**

1. Méthode pour la production d'un GRF recombinant comprenant la croissance d'une bactérie transformée par un vecteur d'expression bactérienne réplicable contenant une séquence d'ADN consistant en un gène de structure codant pour GRF-OH(44) humain avec la séquence d'acides aminés

```
Tyr-Ala-Asp-Ala-Ile-Phe-Thr-Asn-Ser-Tyr-Arg-Lys-Val-Leu-Gly-Gln-
Leu-Ser-Ala-Arg-Lys-Leu-Leu-Gln-Asp-Ile-Met-Ser-Arg-Gln-Gln-Gly-
Glu-Ser-Asn-Gln-Glu-Arg-Gly-Ala-Arg-Ala-Arg-Leu
```

pu pour un fragment biologiquement actif de celui-ci avec au moins les 37 premiers acides aminés et en un site de reconnaissance EcoR I à l'extrémité codant pour l'extrémité amino terminale dudit GRF et un site de reconnaissance Sal I à l'extrémité codant pour l'extrémité carboxy terminale dudit GRF, qui est lié de façon opérationnelle à une séquence d'ADN (promoteur et opérateur) capable d'effectuer l'expression bactérienne dudit GRF-OH, l'induction de l'expression du gène recombinant, la lyse de la bactérie à la fin de la période d'induction et la séparation dudit GRF des constituants cellulaires de ladite bactérie.

2. La méthode selon la revendication 1 dans laquelle le gène de structure code pour GRF-OH(44) humain.

3. La méthode selon la revendication 1 dans lequelle le gène de structure code pour GRF-OH(40) humain.

4. La méthode selon la revendication 1 dans laquelle le gène de structure code pour GRF-OH(37) humain.

5. La méthode de l'une quelconque des revendications 1 à 4, avec un promoteur et un opérateur dérivés du bactériophage lambda et un site de liaison ribosomique hybride.

6. La méthode selon la revendication 5 dans laquelle le promoteur et l'opérateur sont $P_L$ et $O_L$.

7. La méthode de la revendication 6 dans laquelle le vecteur d'expression bactérienne réplicable est le plasmide pRC23 dans lequel, après clivage par EcoR I et Sal I, a été inséré le gène de structure selon la revendication 1.

8. La méthode de l'une quelconque des revendications 1 à 7 dans laquelle la bactérie est une souche de E. coli.

9. La méthode de l'une quelconque des revendications 1 à 8 dans laquelle ladite bactérie est cultivée à une température comprise entre environ 30°C et 36°C et l'expression du gène est induite en élevant la température à environ 42°C.

## Figure I

$P_L$-250

'MODEL' RBS

BglII    HaeIII

TTAAAAATTAAGGAGG
AATTTTTAATTCCTCCTTAA

LIGATE

+BglII
+EcoRI

ISOLATE 265 bp FRAGMENT

LIGATE
+
TRANSFORM

BglII    EcoRI
         BamHI
PstI  ampR   tetR

pRC2

Bg II    EcoRI
         BamHI
         SalI
         $P_L$
PstI  ampR

pRC23

## Figure 2

THE TRANSLATED SEQUENCE IS:

```
              22                    37                    52
ATG TAT GCT GAC GCG ATC TTC ACT AAC TCT TAC CGT AAG GTC CTC GGT CAA
MET TYR ALA ASP ALA ILE PHE THR ASN SER TYR ARG LYS VAL LEU GLY GLN


      67                    82                    97                   112
CTT TCC GCC AGG AAG TTG CTG CAG GAT ATT ATG TCT AGA CAA CAA GGC GAA TCC
LEU SER ALA ARG LYS LEU LEU GLN ASP ILE MET SER ARG GLN GLN GLY GLU SER


          127                 142
AAC CAA GAG CGT GGT GCT CGA GCT CGT TTG TAA GTC GAC G
ASN GLN GLU ARG GLY ALA ARG ALA ARG LEU
```

THE NUCLEOTIDE SEQUENCE IS:

```
    10        20        30        40        50
AATTCTATG TATGCTGACG CGATCTTCAC TAACTCTTAC CGTAAGGTCC
    GATAC ATACGACTGC GCTAGAAGTG ATTGAGAATG GCATTCCAGG


    60        70        80        90       100
TCGGTCAACT TTCCGCCAGG AAGTTGCTGC AGGATATTAT GTCTAGACAA
AGCCAGTTGA AAGGCGGTCC TTCAACGACG TCCTATAATA CAGATCTGTT


   110       120       130       140       150
CAAGGCGAAT CCAACCAAGA GCGTGGTGCT CGAGCTCGTT TGTAAGTCGA
GTTCCGCTTA GGTTGGTTCT CGCACCACGA GCTCGAGCAA ACATTCAGCT


CG
GCTTAA
```

## Figure 3

ARROWS MARK RECOGNITION SITES NOT CUTTING SITES.

```
        10        20        30        40        50        60        70
GAATTCTATGTATGCTGACGCGATCTTCACTAACTCTTACCGTAAGGTCCTCGGTCAACTTTCCGCCAGG
⌢          ⌢⌢  ⌢⌢                     ⌢ ⌢    ⌢          ⌢
EcoRI                DFNI                AEUI   HINDII      EcoRII
EcoRI                FNUDII              AVAII
           HGAI MBDI                     MNLI
                MBDII
```

```
        80        90       100       110       120       130       140
AAGTTGCTGCAGGATATTATGTCTAGACAACAAGGCGAATCCAACCAAGAGCGTGGTGCTCGAGCTCGTT
   ⌢⌢           ⌢          ⌢⌢                 ⌢ ⌢⌢ ⌢⌢
   BBVI          XBAI       EcoRI•             HGI AI   ALUI
   FNU4HI                   HINFI              AVAI
     PSTI                   HINFIII              HGIAI
                                                HGIJII
                                                SACI
                                                TAQI
                                                XHDI
```

```
     150
TGTAAGTCGACGAATTC
   ⌢⌢    ⌢⌢
   AccI    EcoRI
   SALI    EcoRI•
HINDII
       HINFIII
   TAQI
```

Figure 4

GRF-OH(44) Gene Synthesis

| Fragment # | 5'- Sequence -3' | |
|---|---|---|
| 1 | AATTCTATGTATGCTGA | (2-18) |
| 2 | ATCGCGTCAGCATACATAG | (134-152') |
| 3 | CGCGATCTTCACTAACT | (19-35) |
| 4 | GGTAAGAGTTAGTGAAG | (117-133') |
| 5 | CTTACCGTAAGGTCCT | (36-51) |
| 6 | TGACCGAGGACCTTAC | (101-116') |
| 7 | CGGTCAACTTTCCGCC | (52-67) |
| 8 | GCAACTTCCTGGCGGAAAGT | (81-100') |
| 9 | AGGAAGTTGCTGCA | (68-81) |
| 10 | GACATAATATCCTGCA | (65-80') |
| 11 | GGATATTATGTCTAGACAA | (82-100) |
| 12 | ATTCGCCTTGTTGTCTA | (48-64') |
| 13 | CAAGGCGAATCCAACCA | (101-117) |
| 14 | CACGCTCTTGGTTGG | (33-47') |
| 15 | AGAGCGTGGTGCTCGA | (118-133) |
| 16 | ACGAGCTCGAGCAC | (19-32') |
| 17 | GCTCGTTTGTAAGTCGACG | (134-152) |
| 18 | AATTCGTCGACTTACAA | (2-18') |

4

Figure 5

FRAGMENT N̲ (x 2)= 160 bp

## Figure 6

FRAGMENT $\underline{C}$ (x 2) = 140 bp

Figure 7

Figure 8

Solid Support Phosphite Methodology

Loading of the Support

B̂ = Protected base

DMT = Dimethoxytrityl

## Figure 9

Oligodeoxynucleotide Synthesis Scheme

## Figure 10

### Deprotection and Release of Oligonucleotides from the Support

Figure II

B = Thymine (T)

    N – benzoyladenine (A$^{Bz}$)

    N – anisoylcytidine (C$^{An}$)

    N – isobutyrylguanine (G$^{ibu}$)

DMT = Dimethoxytrityl

Figure 12

Figure 13

14'        TACTCTTGGTTGG

15'        AGAGTAAGTCGACG

16'        AATTCGTCGACT

15'

AGAGTAAGTCGACG

GGTTGGTTCTCAT TCAGCTGCTTAA

14'        ↑    16'

Figure 14

14"    CCACGCTCTTGGTTGG

15"    AGAGCGTGGTGCTTAAGTCGACG

16"    AATTCGTCGACTTAAGCA

15"

AGAGCGTGGTGCTTAAGTCGACG

GGTTGGTTCTCGCACCACGAATTCAGCTGCTTAA

14"                              16"

Figure 15